# EUROPEAN PATENT APPLICATION

(11) **EP 1 077 214 A1**
(43) Date of publication of application: **21.02.2001**
(21) Application number: 99116030.0
(22) Date of filing: 16.08.1999
(51) Int. Cl.: C07D 487/04, C07D 231/38, C07D 231/16, C07D 231/14

(54) **An improved process for preparing a pyrazolopyrimidinone derivative**

(71) Applicant: Orchid Chemicals & Pharmaceuticals Ltd., Chennai 600 086 (IN)
(72) Inventor: Chaudhari, Deoram Totaram, 2-A Coral Sands, Chennai 600 090 (IN); Deshpande, Pandurang Balwantrao,, Indira Nagar, Chennai 600 020 (IN); Khan, Rashid Abdul Rehman, K-2 Manek Apt., Adyar, Chennai 600 020 (IN)
(74) Representative: Beneduce, Gianna

(57) **Abstract**

An improved process for the preparation of 5-[2-ethoxy-5-(4-methylpiperazin-1-ylsulphonyl)phenyl]-1-methyl-3-n.propyl-1,6-dihydro-7H-pyrazole[4,3-d]pyrimidin-7-one, which allows to obtain the desired product in good yield and high purity degree, is described.

## Description

The present invention relates to an improved process for the preparation of 5-[2-ethoxy-5-(4-methylpiperazin-1-ylsulphonyl)phenyl]-1-methyl-3-n.propyl-1,6-dihydro-7H-pyrazole[4,3-d]pyrimidin-7-one, a well known compound usefully employed in therapy as vasodilator agent. This compound, generically named sildenafil, is, in particular, endowed with a potent inhibiting effect on specific phosphodiesterases and therefore it has been successfully studied and developed in the treatment of male erectile disfunction.

Another object of the present invention is to provide an improved method for the preparation of 4-amino-1-methyl-3n.propylpyrazole-5-carboxamide, a key intermediate in the synthesis of sildenafil.

A further object of the present invention is to provide an improved method for the preparation of 5-(2-ethoxyphenyl)-1-methyl-3-n.propyl-1,6-dihydro-7H-pyrazole[4,3-d]pyrimidin-7-one which also represents an intermediate in the synthesis of sildenafil.

Sildenafil is described and claimed in USP n°5,250,534 issued on October 5,1993 in the name of the firm Pfizer Inc.: the process therein described consists of a number of steps and provides the desired product in a rather unsatisfactory purity degree and with low yield.

An improved process for the preparation of sildenafil has been lately provided and it forms the object of the co-pending Eur.Appln.n°98122031.2. The process therein described is based on reacting a mixed anhydride of 2-ethoxybenzoic acid with 4-amino-1-methyl-3-n.propylpyrazole-5-carboxamide hydrochloride, treating the so obtained 4-(2-ethoxybenzamido)-1-methyl-3-n.propylpyrazole-5-carboxamide with chlorosulphonic acid and the so obtained corresponding derivative with 1-methylpiperazine to give 4-[2-ethoxy-5-(4-methylpiperazin-1-ylsulphonyl)benzamido]-1-methyl-3-n.propylpyrazole 5-carboxamide which is finally cyclized to 5-[2-ethoxy-5-(4-methylpiperazin-1-ylsulphonyl)phenyl]-1-methyl-3-n.propyl-1,6-dihydro-7H-pyrazole[4,3-d]pyrimidin-7-one.

According to the present invention it is provided a process which, through an improved method for obtaining some important intermediate products of the synthesis, allows to obtain the end product sildenafil in a highly pure degree and with very good yield. Said process further demontrated to be economically advantageous when compared to the known processes.

The process of the present invention is carried out following the reaction scheme hereinafter depicted.

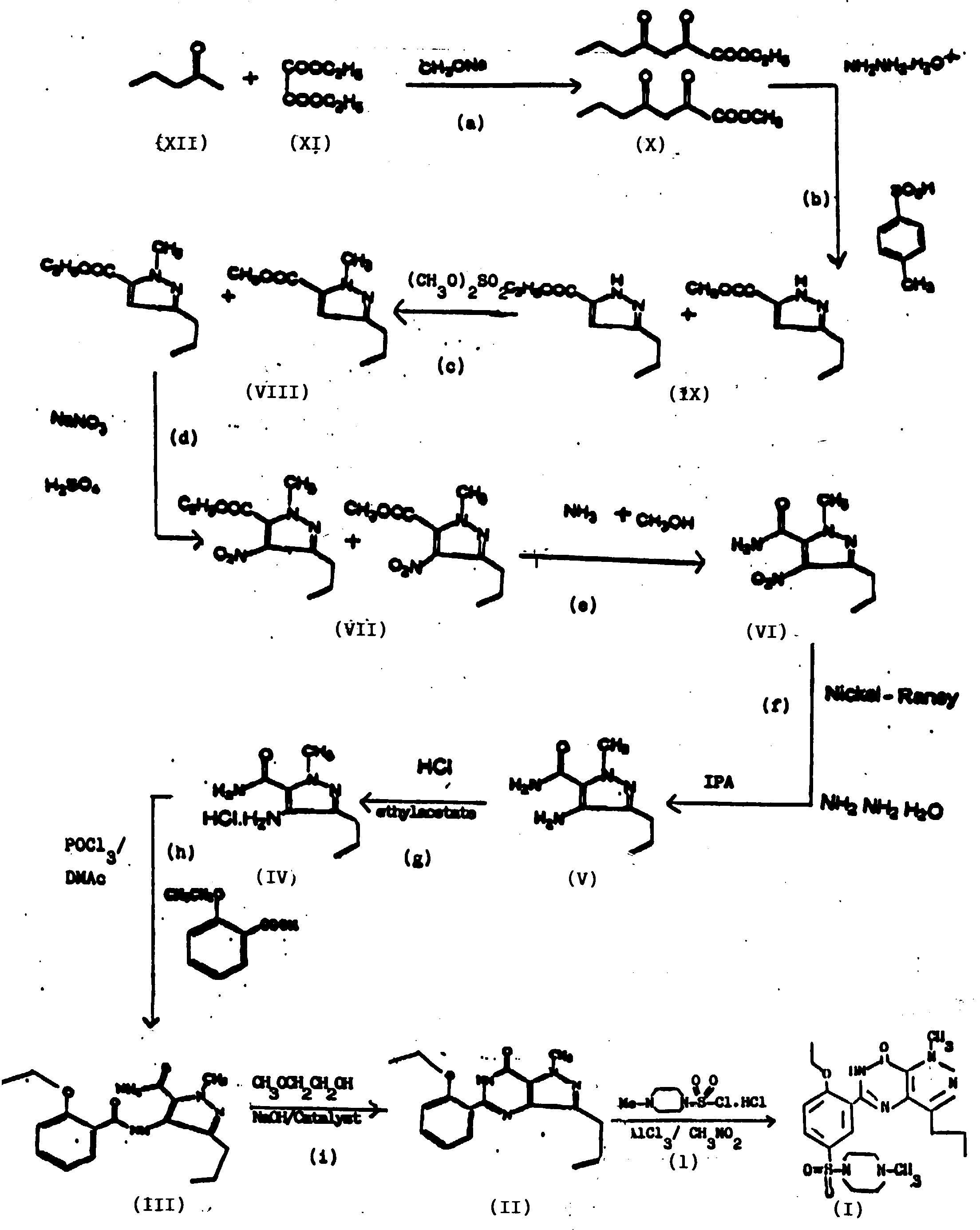

The reaction between the pentanone (XII) and the dioxalate (XI) is carried out in the presence of sodium methoxide and, surprisingly, it gives a mixture of the methyl and ethyl esters of the heptanoic acid (X) which can be successively reacted with hydrazine hydrate and p.toluensulfonic acid to cyclize to the methyl and ethyl esters mixture of the pyrazole acid (IX). The reactions (a) and (b) are carried out in situ without separation and provide the pyrazole derivative (IX) with a yield of 95% much higher when compared to the yield obtained with the known procedure. The nitration reaction (d) performed on the methyl derivative mixture (VIII), obtained by methylation reaction (c) on the pyrazole compound (IX), by means of sodium nitrate and sulphuric acid, represents, in terms of safety, a noticeable improvement in respect to the previous method employing oleum and fuming nitric acid. The reaction (e) by which the amide (VI) forms from the mixture of the methyl and ethyl esters of the pyrazolecarboxylic acid (VII) by carrying out the amidation reaction in anhydrous condition under pressure, also represents an improvement, since in comparison to the previous method it allows avoiding the problems caused by using thionyl chloride and aqueous ammonia. The pyrazoleamide compound (VI) is then reacted (f,g) in the warm in the presence of hydrazine hydrate using as catalyst nickel-Raney and then with hydrochloric acid to give the desired 1-methyl-4-amino-3-n.propylpyrazole-5-carboxamide hydrochloride (IV) in very good purity degree. This latter compound (IV), which is described and claimed per sé in co-pending Eur.Appln. n°98122031.2, is further reacted with 2-ethoxybenzoic acid using phosphorus oxychloride and dimethylacetamide (h) to provide the corresponding diamide derivative (III) which is then cyclized in anhydrous 2-methoxyethanol in the presence of sodium hydroxide and a catalytic amount of tetra.butylammonium bromide (i) to give 5-(2-ethoxyphenyl)-1-methyl-3-n.propyl-1,6-dihydro-7H-pyrazole-[4,3-d]pyrimidin-7-one (II) which is finally made to react in anhydrous conditions with 4-methylpiperazin-1-ylsulfamoyl chloride in the presence of aluminum trichloride (1) to give the desired 5-[2-ethoxy-5-(4-methylpiperazin-1-ylsulphonyl)phenyl]-1-methyl-3-n.propyl-1,6-dihydro-7H-pyrazole [4,3-d] pyrimidin-7-one (I).

The following Examples are given for better illustrating the invention without limiting it.

### Example 1

### Methyl and ethyl esters of 3-n.propylpyrazole-5-carboxylic acid (in admixture)(IX)

Grams 524.00 diethyloxalate and 308.00g 2-pentanone are charged at room temperature in a 51 RBF flask provided with a stirrer, thermowell and condenser. The reaction mixture is cooled to 0/-2°C and 198.00g sodium methoxide are added in four portions of 49.5g each according to the following procedure. The first lot of 49.5g is added, at 0°C, to the mixture maintained over an ice salt bath at about -5/-10°C, then the temperature is allowed to reach 28/30°C and when stabilized, the ice salt bath is removed. The reaction mixture is stirred for 30 minutes and cooled again to 5°C over an ice salt bath. The second lot of 49.5g is added at 5°C to the mixture kept over an ice salt bath at about -5/-10°C. The temperature is allowed to rise up to 30/35°C and when stabilized, the ice salt bath is removed; the mixture is stirred for 30 minutes at 30/35°C and cooled again to 5°C. Addition of the third lot is carried out according to procedure carried out for the second lot: when the reaction temperature is stabilized at 30/35°C the fourth lot is immediately charged thereto and the temperature allowed to rise up to 50/55°C. When the temperature exceeds 50°C, the reaction mixture is cooled over a water bath maintained at 20/25°C and after a period of about 10-15 minutes, when the temperature is stabilized at 55/60°C the cooling is removed and the mixture stirred for 1 hour at the same temperature. The mixture is cooled to 20°C and 1000ml ethylacetate are added thereto, the temperature lowered to 10°C, then 1025ml demineralized water added and stirring is kept till the reaction mixture is quenched in 101 flask and cooled to 5/0°C. The pH of the reaction mixture is adjusted to 1.8-2.0 by adding 720ml of 6N hydrochloric acid solution, stirred for 15 minutes and the layers separated. The aqueous layer is extracted with 230ml ethyl acetate and the obtained organic layers are combined together and washed with 200ml of a 40% saturated brine solution. Grams 15.5 solid p.toluensulfonic acid are added to the organic phase cooled to 5°C, then the reaction temperature is slowly raised to 10°C and 185ml 80% hydrazine hydrate are added in a period of 2 hours, while the temperature is checked not to exceed 22°C. The reaction mixture is then stirred for 30 minutes at 30/32°C, cooled to 5°C, added with 1000ml demineralized water and with a 30% aqueous solution of sodium hydroxide to adjust the pH to 7.2. The layers are separated and the aqueous solution reextracted with 200ml ethylacetate. The combined organic phases are washed with 200ml of a 10% brine solution, concentrated under vacuum till dryness, to obtain 600-616g of a mixture of 3-n.propylpyrazole-5-carboxylic acid methyl and ethyl esters. Yield 95%.

### Example 2

### Methyl and ethyl esters of 1-methyl-3-n.propylpyrazole-5-carboxylic acid (in admixture)(VIII)

To 616g of the mixture of methyl and ethyl esters of 3-n.propylpyrazole-5-carboxylic acid are added, at room temperature and in a period of 1-2 hours, 415g dimethyl sulfate. The reaction temperature is checked not to exceed 58/60°C and it is kept under stirring for 2 hours. Then it is cooled to 20°C, added with 2250ml methylene chloride, 1760ml demineralized water and cooled to 10°C. The pH is adjusted to 7-7.3 by addition of 360ml of a 30% sodium hydroxide solution while the temperature is kept below 15°C. After stirring for 15 minutes the layers are separated: the organic phase, distilled under vacuum at 30/35°C, gives 610-616g of a mixture of 1-methyl-3-n.propylpyrazole-5-carboxylic acid methyl and ethyl esters.

### Example 3

### Methyl and ethyl esters of 1-methyl-4-nitro-3-n.propylpyrazole-5-carboxylic acid (in admixture)(VII)

To 1850ml concentrated sulphuric acid cooled, under stirring, to 15/20°C, 770g sodium nitrate are added and the temperature of the mixture maintained below 40°C. Grams 616 of the mixture of methyl and ethyl esters of 1-methyl-3-n.propylpyrazole-5-carboxylic acid are slowly added thereto checking the temperature does not exceed 50°C. The reaction mixture is heated to 65/70°C, kept under stirring for 6 hours and for a further period at 30-35°C , cooled to 25°C then quenched in 7400ml 5% sodium chloride solution pre-cooled at 0°C and extracted three times with methylene chloride (3080ml+3080ml+1540ml). The combined organic phases are then distilled under vacuum at 40/45°C to give 610-616g of a mixture of 1-methyl-4-nitro-3-n.propylpyrazole-5-carboxylic acid ethyl and methyl esters.

### Example 4

### 1-Methyl-4-nitro-3-n.propylpyrazole-5-carboxamide (VI)

Grams 616 of the mixture of methyl and ethyl esters of 1-methyl-4-nitro-3-n.propylpyrazole-5-carboxylic acid and 2460ml methanol are charged in an autoclave arranged with stirrer, thermowell and auto-cooling system. Ammonia gas is introduced into the autoclave and maintained under the pressure 20-25psi for 2 hours while keeping the temperature at 50°C by means of a water bath.

The reaction mixture is then cooled to 30/25°C, poured into 101 pre-cooled demineralised water and stirred at 0°C for 30 minutes. The product obtained is filtered, washed with 2326ml cold demineralised water and dried under vacuum at room temperature to give 360-370g 1-methyl-4-nitro-3-n.propylpyrazole-5-carboxamide, melting at 142-143°C.

### Example 5

### 1-Methyl-4-amino-3-n.propylpyrazole-5-carboxamide hydrochloride (IV)

In a 31 flask arranged with a water bath with stirrer, thermowell, condenser and nitrogen gas connection, 16.5ml nickel-Raney catalyst, 450ml isopropylalcohol (IPA) and 150g 1-methyl-4-nitro-3-n.propylpyrazole-5-carboxamide and further 150ml isopropylalcohol are charged and slowly heated to reflux. In a period of an hour, an hour and a half, 110ml of an hydrazine hydrate solution are added thereto dropwise, maintained under mild reflux for 30 minutes (80°C) and then cooled to room temperature. The catalyst is removed by filtration under nitrogen atmosphere and washed with 300ml isopropylalcohol. The isopropylalcohol is removed from the mixture by distillation under vacuum, at 45°C, of the reaction mixture and 170g of a residue is obtained, which is treated with 300ml water, 7.5g activated charcoal and 3.7g sodium metabisulfite, stirred for 30 minutes and the carbon removed by filtration. The water solution is extracted four times with 300ml each ethylacetate and the combined organic phases added with 7.5g activated charcoal, stirred at 40/45°C for 30 minutes and then filtered. To the clear filtrate obtained 75ml concentrated hydrochloric acid are added at room temperature in a period of 1 hour and the precipitate stirred at room temperature for 30 minutes. The precipitate is dried, washed with 600ml ethylacetate, twice (1200ml+300ml) with dry acetone and again dried at room temperature on a tray drier to give 105g 1-methyl-4-amino-3-n.propylpyrazole-5-carboxamide hydrochloride, melting at 240-242°C. Purity 97.5-98%. Yield 35%.
¹H-NMR at 200MHz in DMSO D₆
8.5-8.3 ppm bs[2H]exch D₂O (H_{a+b})
3.87 ppm s[3H] (H_{c})
2.58 ppm t[2H] (J=7.5 Hz) (H_{d})
1.53 ppm hex[2H] (J=7.4 Hz) (Hₑ)
0.88 ppm t[3H] (J=7.3 Hz) (H_{f})
C-NMR at 200MHz in DMSO
159.757 ppm [C]
144.496 ppm [C]
131.857 ppm [C]
38.799 ppm [CH₃]
26.449 ppm [CH₂]
22.219 ppm [CH₂]
13.627 ppm [CH₃]

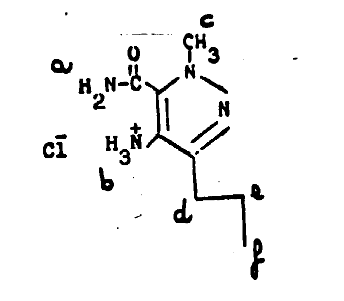

### Example 6

### 4-(2-Ethoxybenzamido)-1-methyl-3-n.propylpyrazole-5-carboxamide (III)

To 500ml dimethylacetamide charged in a 21 flask are added under stirring 95.1g 2-ethoxy benzoic acid and, after cooling to -5°C, slowly, over a 20 minute period, 95.5g phosphorous oxychloride keeping the temperature below 5°C and stirring for 1 further hour. Grams 100 1-methyl-4-amino-3-n.propylpyrazole-5-carboxamide hydrochloride are added thereto and the reaction mixture is first warmed to 25/30°C and stirred for 90-100 minutes, then cooled to 5°C, added with 1000ml demineralised water and pH adjusted to 5 by adding an aqueous sodium hydroxide solution. A colorless crystalline product precipitates which is filtered, washed with 200ml demineralised water and dried under vacuum at 60°C, to give 138-142g 4-(2-ethoxybenzamido)-1-methyl-3-n.propylpyrazole-5-carboxamide, melting at 155°C. Purity >98.5%.
I.R. (Nujol -cm-1) 3447.6; 3351.3; 3175.1; 1645.4; 1611.8; 1555.2
¹H-NMR at 200 MHz in DMSO D₆
9.51 ppm s[1H] (H₅)
7.78 ppm bs[1H] (H₁₂)
7.66 ppm d[1H] (J = 7.5 Hz) (H₆)
7.50 ppm t[1H] (J = 7.8 Hz) (H₈)
7.39 ppm bs[1H] (H₁₃)
7.18 ppm d[1H] (J = 8.3 Hz) (H₉)
7.06 ppm t[1H] (J = 7.4 Hz) (H₇)
4.20 ppm q[2H] (J = 7.0 Hz) (H₁₀)
3.93 ppm s[3H] (H₁)
2.46 ppm t[2H] (J = 7.6 Hz) (H₂)
1.59 ppm sext[2H] (J = 7.4 Hz) (H₃)
1.40 ppm t[3H] (J = 6.9 Hz) (H₁₁)
0.89 ppm t[3H] (J = 7.4 Hz) (H₄)

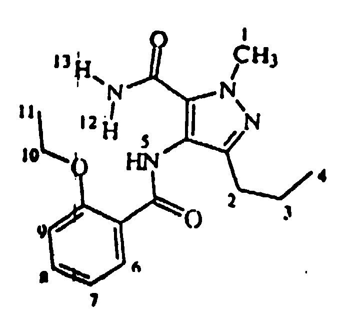

### Example 7

### 5-(2-Ethoxyphenyl)-1-methyl-3-n.propyl-1,6-dihydro-7H-pyrazole[4,3-d]pyrimidin-7-one (II)

To 325ml 2-methoxyethanol are added, under stirring, 14.6g sodium hydroxide, heated to 50/55°C till complete dissolution, then 100g 4-(2-ethoxybenzamido)-1-methyl-3-n.propylpyrazole-5-carboxamide and lg tetra.butylammonium bromide are added thereto under stirring. The mixture is rinsed with 25ml 2-methoxyethanol, heated to 105/110°C, then after 45-60 minutes at this temperature, it is cooled to 50°C and poured into 2000ml demineralised water at 5°C. Add slowly at 15/20°C 80ml 1:1 hydrochloric acid till pH 1.5 and keep under stirring for 1 hour. The product is separated by filtration, washed with 500ml demineralised water, dried under vacuum at 60°C to give 90-92g 5-(2-ethoxyphenyl)-1-methyl-3-n.propyl-1,6-dihydro-7H-pyrazole[4,3-d]pyrimidin-7-one, melting at 145.4°C. Purity >98.5%.

### Example 8

### 5-[2-ethoxy-5-(4-methylpiperazin-1-ylsulphonyl)phenyl]-1-methyl-3-n.propyl-1,6-dihydro-7H-pyrazole[4,3-d]pyrimidin-7-one (I)

To a mixture of 50 ml anhydrous nitromethane and 5 g (16 mmoles) 5-(2-ethoxyphenyl)-1-methyl-3-n.propyl-1,6-dihydro-7H-pyrazole[4,3-d]pyrimidin-7-one, at room temperature, 3.2 g (24 mmoles) of granular anhydrous AlCl₃ in small portions, were added, and subsequently, under efficient stirring 4.14 g (17.6 mmoles) 4-methylpiperazin-1-ylsulfamoylchloride hydrochloride [prepared according to W.L. Matier, J.Med.Chem.15,538(1972)]. The reaction mixture was refluxed for 5 to 7 hours, then cooled and submitted to evaporation to remove the solvent. The residue was taken up with 200 ml methylene chloride and 150 ml concentrated hydrochloric acid; after 10 minutes under stirring the two phases were separated and the pH of the aqueous layer was adjusted to 12 using 40% aqueous NaOH solution. After 15 minutes of stirring the clear solution obtained was carefully treated with 5M hydrochloric acid to adjust the pH to 7.

The insoluble material was removed by filtration, washed with methylene chloride and the combined washings were used to exhaustively extract the aqueous filtrate. The organic phase was separated, treated with charcoal and, after filtration, evaporated under vacuum to give a solid residue that was then dissolved in water at pH 13, filtered in the presence of charcoal and precipitated adjusting the pH to 9.25 with 1M hydrochloric acid. The precipitate was filtered, washed with water at 10°C and dried under vacuum at 40°C to give 3.41 g 5-[2-ethoxy-5-(4-methylpiperazin-1-ylsulfonyl)phenyl]-1-methyl-3-n-propyl-1,6-dihydro-7H-pyrazolo[4,3-d]pyrimidin-7-one, melting at 187-189°C.
¹H-NMR at 200 MHz in DMSO D₆
12.20 ppm bs[1H] (exch D₂O) (H₁₃)
7.88-7.79 ppm m[2H] (H₅, H₆)
7.37 ppm d[1H] (H₇)
4.21 ppm q[2H] (H₈)
4.16 ppm s[3H] (H₁)
2.91 ppm bt[4H] (H₁₀)
2.77 ppm t[2H] (J = 7.4 Hz) (H₂)
2.36 ppm t[4H] (J = 4.6 Hz) (H₁₁)
2.14 ppm s[3H] (H₁₂)
1.73 ppm sext[2H] (J = 7.3 Hz) (H₃)
1.33 ppm t[3H] (J = 6.8 Hz) (H₉)
0.93 ppm t[3H] (J = 7.3 Hz) (H₄)
¹H NMR at 200MHz in CDCl₃
10.82 ppm bs[1H] exch. D₂O (H₁₃)
8.83 ppm d[1H] (J= 2.2Hz) (H₅)
7.84 ppm dd[1H] (J= 8.7Hz J2=2.4Hz) (H₆)
7.15 ppm d[1H] (J= 8.7Hz) (H₇)
4.38 ppm q[2H] (J= 6.9Hz) (H₈)
4.28 ppm s[3H] (H₁)
3.12 ppm bt[4H] (J=4.6Hz) (H₁₀)
2.93 ppm t[2H] (J=7.5Hz) (H₂)
2.51 ppm t[4H] (J=4.8Hz) (H₁₁)
2.28 ppm s[3H] (H₁₂)
1.87 ppm sext[2H] (J=7.4Hz) (H₃)
1.65 ppm t[3H] (J=6.9Hz) (H₉)
1.03 ppm t[3H] (j=7.3Hz) (H₄)

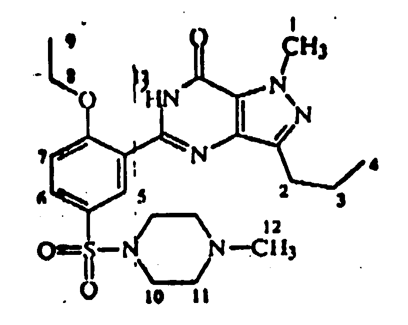

## Claims

1. A process for the preparation of 5-[2-ethoxy-5-(4-methylpiperazin-1-ylsulphonyl)phenyl]-1-methyl-3-n.propyl-1,6-dihydro-7H-pyrazole[4,3-d]pyrimidin-7-one, characterized in that diethyloxalate is reacted with 2-pentanone in the presence of sodium methoxide at a temperature ranging from 35 to -15°C and that the mixture of the methyl and ethyl esters of the 2,4-dioxoheptanoic acid obtained is directly treated with hydrazine hydrate in the presence of p.toluensulfonic acid to give a mixture of the methyl and ethyl esters of 3-n.propylpyrazole-5-carboxylic acid which is reacted with dimethylsulphate and that the corresponding mixture of esters of 1-methylderivative obtained is reacted with sodium nitrate in the presence of sulphuric acid to provide the mixture of methyl and ethyl esters of 1-methyl-4-nitro-3-n.propylpyrazole-5-carboxylic acid which is treated with ammonia under pressure at a temperature ranging from 45 to 60°C in a suitable solvent and that 1-methyl-4-nitro-3-n.propylpyrazole-5-carboxamide so obtained is reduced under pressure with nickel-Raney and hydrazine hydrate to give the 4-amino-1-methyl-3n.propylpyrazole-5-carboxamide which is suitably treated with hydrochloric acid to give the corresponding hydrochloride addition salt which is reacted with 2-ethoxybenzoic acid using phosphorus oxychloride and dimethylacetamide and that 4-(2-ethoxybenzamido)-1-methyl-3-n.propylpyrazole-5-carboxamide is then cyclized in anhydrous 2-methoxyethanol in the presence of sodium hydroxide and a catalytic amount of tetra.butylammonium bromide and that the obtained 5-(2-ethoxyphenyl)-1-methyl-3-n.propyl-1,6-dihydro-7H-pyrazole-[4,3-d]pyrimidin-7-one is reacted in anhydrous conditions with 4-methylpiperazin-1-sulfamoyl chloride in the presence of aluminum trichloride to give 5-[2-ethoxy-5-(4-methylpiperazin-1-ylsulphonyl)phenyl)-1-methyl-3-n.propyl-1,6-dihydro-7H-pyrazole[4,3-d]pyrimidin-7-one.

2. A process for the preparation of 4-amino-1-methyl-3-n.propylpyrazole-5-carboxamide hydrochloride characterized in that diethyloxalate is reacted with 2-pentanone in the presence of sodium methoxide at a temperature ranging from 35 to -15°C and that the mixture of the methyl and ethyl esters of the 2,4-dioxoheptanoic acid obtained is directly treated with hydrazine hydrate in the presence of p.toluensulfonic acid to give a mixture of the methyl and ethyl esters of 3-n.propylpyrazole-5-carboxylic acid which is reacted with dimethylsulphate and that the corresponding mixture of esters of 1-methyl derivative obtained is reacted with sodium nitrate in the presence of sulphuric acid to provide the mixture of methyl and ethyl esters of 1-methyl-4-nitro-3-n.propylpyrazole-5-carboxylic acid which is treated with ammonia under pressure at a temperature ranging from 45 to 60°C in a suitable solvent and that 1-methyl-4-nitro-3-n.propylpyrazole-5-carboxamide so obtained is reduced under pressure with nickel-Raney and hydrazine hydrate to give the 4-amino-1-methyl-3n.propylpyrazole-5-carboxamide which is suitably treated with hydrochloric acid to give the corresponding hydrochloride addition salt.

3. A process for the preparation of 5-[2-ethoxyphenyl]-1-methyl-3-n.propyl-1,6-dihydro-7H-pyrazole[4,3-d]pyrimidin-7-one, characterized in that diethyloxalate is reacted with 2-pentanone in the presence of sodium methoxide at a temperature ranging from 35 to -15°C and that the mixture of the methyl and ethyl esters of the 2,4-dioxoheptanoic acid obtained is directly treated with hydrazine hydrate in the presence of p.toluensulfonic acid to give a mixture of the methyl and ethyl esters of 3-n.propylpyrazole-5-carboxylic acid which is reacted with dimethylsulphate and that the corresponding mixture of esters of 1-methylderivative obtained is reacted with sodium nitrate in the presence of sulphuric acid to provide the mixture of methyl and ethyl esters of 1-methyl-4-nitro-3-n.propylpyrazole-5-carboxylic acid which is treated with ammonia under pressure at a temperature ranging from 45 to 60°C in a suitable solvent and that 1-methyl-4-nitro-3-n.propylpyrazole-5-carboxamide so obtained is reduced under pressure with nickel-Raney and hydrazine hydrate to give the 4-amino-1-methyl-3n.propylpyrazole-5-carboxamide which is suitably treated with hydrochloric acid to give the corresponding hydrochloride addition salt which is reacted with 2-ethoxybenzoic acid using phosphorus oxychloride and dimethylacetamide and that 4-(2-ethoxybenzamido)-1-methyl-3-n.propylpyrazole-5-carboxamide is cyclized in anhydrous 2-methoxyethanol in the presence of sodium hydroxide and a catalytic amount of tetra.butylammonium bromide to give 5-(2-ethoxyphenyl)-1-methyl-3-n.propyl-1,6-dihydro-7H-pyrazole-[4,3-d]pyrimidin-7-one.
